# EUROPEAN PATENT SPECIFICATION

(11) **EP 2 351 851 B1**
(45) Date of publication and mention of the grant of the patent: **08.07.2015**
(21) Application number: 09829212.1
(22) Date of filing: 30.11.2009
(51) Int. Cl.: C12Q 1/68, C12N 15/09

(54) **METHOD FOR MEASURING CYTOKERATIN-19 MRNA**
VERFAHREN ZUR CYTOKERATIN-19-MRNA-MESSUNG
PROCÉDÉ POUR MESURER L'ARNM DE CYTOKÉRATINE-19

(30) Priority: 28.11.2008 JP 2008303637
(43) Date of publication of application: 03.08.2011
(73) Proprietor: Tosoh Corporation, Yamaguchi 746-8501 (JP)
(72) Inventor: OMOTO, Daisuke, Ayase-shi Kanagawa 252-1123 (JP); SAITO, Juichi, Ayase-shi Kanagawa 252-1123 (JP); OONAKA, Satoru, Ayase-shi Kanagawa 252-1123 (JP); HAYASHI, Toshinori, Ayase-shi Kanagawa 252-1123 (JP)
(74) Representative: Kügele, Bernhard
(86) International application number: PCT/JP2009/070439
(87) International publication number: WO 2010/061981

(56) References cited:
- EP-A1- 1 475 445
- EP-A1- 1 783 232
- EP-A2- 0 855 447
- EP-A2- 1 780 289
- WO-A1-96/17079
- WO-A2-01/73031
- WO-A2-03/026584
- JP-A- 2000 014 400
- JP-A- 2001 333 783
- JP-A- 2002 320 481
- JP-A- 2003 033 182
- JP-A- 2003 289 869
- JP-A- 2007 275 016
- JP-A- 2008 000 112
- JP-A- 2008 194 028
- US-B1- 6 504 010
- Premierbiosoft: "NASBA Technology", , 1 January 1994 (1994-01-01), XP055004958, Retrieved from the Internet: URL:http://www.premierbiosoft.com/tech_not es/NASBA.html [retrieved on 2011-08-17]
- KIEVITS T ET AL: "NASBA TM ISOTHERMAL ENZYMATIC IN VITRO NUCLEIC ACID AMPLIFICATION OPTIMIZED FOR THE DIAGNOSIS OF HIV-1 INFECTION", JOURNAL OF VIROLOGICAL METHODS, ELSEVIER BV, NL, vol. 35, no. 3, 1 December 1991 (1991-12-01), pages 273-286, XP000576430, ISSN: 0166-0934, DOI: 10.1016/0166-0934(91)90069-C
- ISHIGURO T ET AL: "INTERCALATION ACTIVATING FLUORESCENCE DNA PROBE AND ITS APPLICATION TO HOMOGENEOUS QUANTIFICATION OF A TARGET SEQUENCE BY ISOTHERMAL SEQUENCE AMPLIFICATION IN A CLOSED VESSEL", ANALYTICAL BIOCHEMISTRY, ACADEMIC PRESS INC, NEW YORK, vol. 314, 1 January 2003 (2003-01-01), pages 77-86, XP001165733, ISSN: 0003-2697, DOI: 10.1016/S0003-2697(02)00618-8
- LAMBRECHTS A C ET AL: "COMPARISON OF IMMUNOCYTOCHEMISTRY, REVERSE TRANSCRIPTASE POLYMERASE CHAIN REACTION, AND NUCLEIC ACID SEQUENCE-BASED AMPLIFICATION FOR THE DETECTION OF CIRCULATING BREAST CANCER CELLS", BREAST CANCER RESEARCH AND TREATMENT, SPRINGER, NEW YORK, NY, vol. 56, no. 3, 1 August 1999 (1999-08-01) , pages 219-231, XP009035704, ISSN: 0167-6806, DOI: 10.1023/A:1006261731125
- ISHIGURO, T. ET AL.: 'Intercalation activating fluorescence DNA probe and its application to homogeneous quantification of a target sequence by isothermal sequence amplification in a closed vessel.' ANALYTICAL BIOCHEMISTRY vol. 314, 2003, pages 77 - 86, XP001165733
- OMOTO, D. ET AL.: 'TRC-ho ni yoru Nyugan Sentinel Lymph-setsu Ten'i no Shindan no Yuyosei' THE JAPAN SOCIETY FOR CLINICAL LABORATORY AUTOMATION DAI 41 KAI TAIKAI SHOROKUSHU 01 September 2009, page 578, XP008147159
- OMOTO, D. ET AL.: 'Nyugan Sentinel Lymph-setsu Seiken ni Okeru TRC CK19 mRNA Sokutei no Yuyosei' DAI 56 KAI JAPANESE SOCIETY OF LABORATORY MEDICINE GAKUJUTSU SHUKAI 28 July 2009, page 296, XP008148249

### SEQUENCE LISTING

<110> TOSOH CORPORATION
<120> Method for Measuring Cytokeratin 19 mRNA
<130> W742-PCT
<150> JP2008-303637
   <151> 2008-11-28
<160> 47
<210> 1
   <211> 83
   <212> DNA
   <213> Artificial Sequence
<220>
   <223> CKF1
<400> 1
   ccagaagcag gggcctgggc cctcccgcga ctacagccac tactacacga ccatccagga 60
   cctgcgggac aagattcttg gtg 83
<210> 2
   <211> 30
   <212> DNA
   <213> Artificial Sequence
<220>
   <223> CKF2
<400> 2
   aaccatgagg aggaaatcag tacgctgagg 30
<210> 3
   <211> 29
   <212> DNA
   <213> Artificial Sequence
<220>
   <223> CKF3
<400> 3
   caccagccgg actgaagaat tgaaccggg 29
<210> 4
   <211> 23
   <212> DNA
   <213> Artificial Sequence
<220>
   <223> CKF4
<400> 4
   cacagctgag catgaaagct gcc 23
<210> 5
   <211> 58
   <212> DNA
   <213> Artificial Sequence
<220>
   <223> CKR1
<400> 5
   gttgatgtcg gcctccacgc tcatgcgcag agcctgttcc gtctcaaact tggttcgg 58
<210> 6
   <211> 27
   <212> DNA
   <213> Artificial Sequence
   z
<223> CKR2
<400> 6
   cggttcaatt cttcagtccg gctggtg 27
<210> 7
   <211> 24
   <212> DNA
   <213> Artificial Sequence
<220>
   <223> CKR3
<400> 7
   ccaaggcagc tttcatgctc agct 24
<210> 8
   <211> 47
   <212> DNA
   <213> Artificial Sequence
<220>
   <223> CKR4
<400> 8
   acttgatgtc catgagccgc tggtactcct gattctgccg ctcacta 47
<210> 9
   <211> 23
   <212> DNA
   <213> Artificial Sequence
<220>
   <223> CKS1
<400> 9
   gcttctggta ccagtcgcgg atc 23
<210> 10
   <211> 23
   <212> DNA
   <213> Artificial Sequence
<220>
   <223> CKS2
<400> 10
   tgtagtcgcg ggagggccca ggc 23
<210> 11
   <211> 23
   <212> DNA
   <213> Artificial Sequence
<220>
   <223> CKS3
<400> 11
   cgcaggtcct ggatggtcgt gta 23
<210> 12
   <211> 23
   <212> DNA
   <213> Artificial Sequence
<220>
   <223> CKS4
<400> 12
   tgtcccgcag gtcctggatg gtc 23
<210> 13
   <211> 23
   <212> DNA
   <213> Artificial Sequence
<220>
   <223> CKS5
<400> 13
   tcatggttct tcttcaggta ggc 23
<210> 14
   <211> 23
   <212> DNA
   <213> Artificial Sequence
<220>
   <223> CKS6
<400> 14
   ttcctcctca tggttcttct tca 23
<210> 15
   <211> 23
   <212> DNA
   <213> Artificial Sequence
<220>
   <223> CKS7
<400> 15
   ggctggtgaa ccaggcttca gca 23
<210> 16
   <211> 23
   <212> DNA
   <213> Artificial Sequence
<220>
   <223> CKS8
<400> 16
   ttcagtccgg ctggtgaacc agg 23
<210> 17
   <211> 23
   <212> DNA
   <213> Artificial Sequence
<220>
   <223> CKS9
<400> 17
   cagctgtgac tgcagctcaa tct 23
<210> 18
   <211> 23
   <212> DNA
   <213> Artificial Sequence
<220>
   <223> CKS10
<400> 18
   gctcagctgt gactgcagct caa 23
<210> 19
   <211> 20
   <212> DNA
   <213> Artificial Sequence
<220>
   <223> CKF5
<400> 19
   ccagaagcag gggcctgggc 20
<210> 20
   <211> 25
   <212> DNA
   <213> Artificial Sequence
<220>
   <223> CKF6
<400> 20
   cgactacagc cactactaca cgacc 25
<210> 21
   <211> 21
   <212> DNA
   <213> Artificial Sequence
<220>
   <223> CKF7
<400> 21
   gacctgcggg acaagattct t 21
<210> 22
   <211> 20
   <212> DNA
   <213> Artificial Sequence
<220>
   <223> CKF8
<400> 22
   gcgggacaag attcttggtg 20
<210> 23
   <211> 24
   <212> DNA
   <213> Artificial Sequence
<220>
   <223> CKF9
<400> 23
   aaccatgagg aggaaatcag tacg 24
<210> 24
   <211> 23
   <212> DNA
   <213> Artificial Sequence
<220>
   <223> CKF10
<400> 24
   aggaggaaat cagtacgctg agg 23
<210> 25
   <211> 20
   <212> DNA
   <213> Artificial Sequence
<220>
   <223> CKF11
<400> 25
   caccagccgg actgaagaat 20
<210> 26
   <211> 21
   <212> DNA
   <213> Artificial Sequence
<220>
   <223> CKF12
<400> 26
   ggactgaaga attgaaccgg g 21
<210> 27
   <211> 21
   <212> DNA
   <213> Artificial Sequence
<220>
   <223> CKF13
<400> 27
   cacagctgag catgaaagct g 21
<210> 28
   <211> 20
   <212> DNA
   <213> Artificial Sequence
<220>
   <223> CKF14
<400> 28
   agctgagcat gaaagctgcc 20
<210> 29
   <211> 20
   <212> DNA
   <213> Artificial Sequence
<220>
   <223> CKR5
<400> 29
   ccgtctcaaa cttggttcgg 20
<210> 30
   <211> 22
   <212> DNA
   <213> Artificial Sequence
<220>
   <223> CKR6
<400> 30
   cctgttccgt ctcaaacttg gt 22
<210> 31
   <211> 20
   <212> DNA
   <213> Artificial Sequence
<220>
   <223> CKR7
<400> 31
   tgtcggcctc cacgctcatg 20
<210> 32
   <211> 21
   <212> DNA
   <213> Artificial Sequence
<220>
   <223> CKR8
<400> 32
   gttgatgtcg gcctccacgc t 21
<210> 33
   <211> 20
   <212> DNA
   <213> Artificial Sequence
<220>
   <223> CKR9
<400> 33
   attcttcagt ccggctggtg 20
<210> 34
   <211> 21
   <212> DNA
   <213> Artificial Sequence
<220>
   <223> CKR10
<400> 34
   cggttcaatt cttcagtccg g 21
<210> 35
   <211> 20
   <212> DNA
   <213> Artificial Sequence
<220>
   <223> CKR11
<400> 35
   ggcagctttc atgctcagct 20
<210> 36
   <211> 20
   <212> DNA
   <213> Artificial Sequence
<220>
   <223> CKR12
<400> 36
   ccaaggcagc tttcatgctc 20
<210> 37
   <211> 21
   <212> DNA
   <213> Artificial Sequence
<220>
   <223> CKR13
<400> 37
   tcctgattct gccgctcact a 21
<210> 38
   <211> 20
   <212> DNA
   <213> Artificial Sequence
<220>
   <223> CKR14
<400> 38
   acttgatgtc catgagccgc 20
<210> 39
   <211> 19
   <212> DNA
   <213> Artificial Sequence
<220>
   <223> CKINAF1
<400> 39
   aagaatcttg tcccgcagg 19
<210> 40
   <211> 22
   <212> DNA
   <213> Artificial Sequence
<220>
   <223> CKINAF2
<400> 40
   caggacaatc ctggagttct ca 22
<210> 41
   <211> 24
   <212> DNA
   <213> Artificial Sequence
<220>
   <223> CKINAF3
<400> 41
   tgttccgtct caaacttggt tcgg 24
<210> 42
   <211> 21
   <212> DNA
   <213> Artificial Sequence
<220>
   <223> CKINAF4
<400> 42
   gagatcggtg cccggagcgg a 21
<210> 43
   <211> 21
   <212> DNA
   <213> Artificial Sequence
<220>
   <223> CKINAF5
<400> 43
   ttggcttcgc atgtcactca g 21
<210> 44
   <211> 20
   <212> DNA
   <213> Artificial Sequence
<220>
   <223> CKINAF6
<400> 44
   cctcggacct gctcatctgg 20
<210> 45
   <211> 22
   <212> DNA
   <213> Artificial Sequence
<220>
   <223> CKINAF7
<400> 45
   aagggtgcgc cgcaggtcag ta 22
<210> 46
   <211> 20
   <212> DNA
   <213> Artificial Sequence
<220>
   <223> CKINAF8
<400> 46
   ccgtttctgc cagtgtgtct 20
<210> 47
   <211> 28
   <212> DNA
   <213> Artificial Sequence
<220>
   <223> T7 promoter
<400> 47
   aattctaata cgactcacta tagggaga 28

## Description

### TECHNICAL FIELD

The present invention relates to a method of measuring cytokeratin 19 mRNA using a nucleic acid amplification method. More precisely, the present invention provides an oligonucleotide suitable for amplification and detection of mRNA at a constant temperature (40 to 60°C, and preferably 43°C), and enables cytokeratin 19 mRNA present in a sample to be measured easily and rapidly.

### BACKGROUND ART

Cytokeratin is an intermediate-diameter filamentous protein that forms the cytoskeleton, and is characteristically present in epithelial tissue. More than 20 types of cytokeratin have currently been identified, and are classified based on their protein charge, with acidic cytokeratin classified as type I and neutral to basic cytokeratin classified as type II. The expression site of cytokeratin varies according to the type, with cytokeratin 8, 18 and 19 known to be predominantly expressed in epithelial cells, while cytokeratin 5 and 14 are known to be localized in basal cells, and cytokeratin 2, 6, 10 and 11 are known to be localized in suprabasal tissue.

Cytokeratin is widely recognized in the fields of medicine and other fields due to frequent use in immunostaining using anti-cytokeratin antibody. In many cases, cancer is diagnosed pathologically, and specimens are typically used in which tissue or cells in which cancer is suspected are stained with hematoxylin-eosin (HE) stain. However, sometime it is difficult to distinguish between cancer cells and normal cells in the case of HE staining, resulting in the discovery of cancer cells being overlooked. Therefore, immunohistostaining using anti-cytokeratin antibody is frequently used. As a result of immunohistostaining a specimen other than epithelial tissue using anti-cytokeratin antibody, since a positive reaction indicates the expression of cytokeratin in that tissue even though it only ought to be expressed in epithelial tissue, the tissue is determined to be histologically abnormal, or in other words, is pathologically cancerous.

Although cytokeratin 19 (hereafter, abbreviated as "CK19") is known to not only be predominantly expressed in epithelial tissue, but also in other normal tissue in small amounts, its expression has been clearly determined to be increased in various cancers. Antibodies that recognize CK19 as antigen are frequently used in the immunostaining using anti-cytokeratin antibody described above. These expression characteristics of CK19 satisfy conditions for use as a tumor marker, and CK19 is in fact widely used as a tumor marker. Since expression of CK19 increases in various cancers, it is indicated for numerous types of cancers, its method of use is quite diverse, ranging from early cancer diagnosis and diagnosis of metastasis to monitoring of therapeutic efficacy, and it is considered to be an extremely useful tumor marker.

Although diagnosis of cancer by pathological diagnosis as previously described is widely used and considered to be the gold standard for diagnosing cancer, it requires a high degree of skill on the part of the pathologist, and the potential for overlooking cancer cells has been indicated depending on the specimen used. In recent years, gene testing (nucleic acid amplification) has been proposed or is being used practically at some facilities for the purpose of reducing this potential for overlooking cancer cells or enabling uniform diagnoses between hospitals and other health care facilities. Nucleic acid amplification is already known to have high sensitivity, allows the obtaining of high specificity since it amplifies a specific gene, and enables a specific gene in a sample to be quantified depending on the measurement method. In other words, nucleic acid amplification provides information relating to the magnitude of an expressed amount of a specific gene.

In general, the reverse transcription-polymerase chain reaction (RT-PCR) is widely used as a gene amplification process when detecting tumor marker gene mRNA using nucleic acid amplification, and its application to CK19 mRNA has also been reported (refer to Anne-Marie, C. et al., Laboratory Investigation, 77, 213-220 (1997) (Non-Patent Document 1); Tao, L. et al., British Journal of Cancer, 94, 1164-1169 (2006) (Non-Patent Document 2); Wang, H.Y. et al., International Journal of Gynecological Cancer, 16, 643-648 (2006) (Non-Patent Document 3); Weiggelt, B. et al., British Journal of Cancer, 90, 1531-1537 (2004) (Non-Patent Document 4); Kamiya, M. et al., British Journal of Dermatology, 149, 998-1005 (2003) (Non-Patent Document 5); Makino, H. et al., Hepato-Gastroenterology, 50, 1407-1410 (2003) (Non-Patent Document 6); Bustin, S.A. et al., British Journal of Cancer, 79, 1813-1820 (1999) (Non-Patent Document 7); Fujita, Y. et al., Gastric Cancer, 9, 308-314 (2006) (Non-Patent Document 8); Japanese Translation of PCT International Application Publication No. 2003-527098 (Patent Document 2); Japanese Translation of PCT International Application Publication No. 2005-522231 (Patent Document 3); and, Japanese Translation of PCT International Application Publication No. 2008-502330 (Patent Document 4)). Since nucleic acid amplification requires two steps indicated below following extraction of RNA from tumor tissue, i.e.,
(a) a step for synthesizing cDNA from the extracted RNA with reverse transcriptase, and
(b) a step for detecting the cDNA by amplifying by PCR,
(and it may be necessary to separately carry out an additional detection step depending on the case), complexity of the procedure and the risk of secondary infection are suggested. In addition, since it normally requires 2 hours or more to carry out the two steps, there have been problems with respect to poor reproducibility attributable to carrying out multiple steps as well as reducing large-volume specimen processing and testing costs. Although a One Step RT-PCR method has been developed that enables both steps to be carried out simultaneously in order to shorten reaction time, this method has been indicated as having lower detection sensitivity and resulting in the production of non-specific amplification products in comparison with RT-PCR methods in which each step is performed separately. Moreover, since amplification by PCR involves amplification of double-stranded DNA, there is concern over the possibility of amplifying contaminating chromosomal DNA, thereby resulting in the need to completely remove all chromosomal DNA by digesting with DNase and the like in order to precisely analyze mRNA expression. Consequently, this results in greater complexity of the procedure and poorer reproducibility. Moreover, since the PCR method requires the reaction temperature to be rapidly raised and lowered, it serves as an obstacle to labor saving and cost reduction of reaction devices at the time of automation.

In addition, amplification and detection of CK19 mRNA has also been reported that uses a different nucleic acid amplification process in the form of Reverse Transcription-Loop-mediated isothermal AMPlification (RT-LAMP) (refer to Tujimoto, M. et al., Clinical Cancer Research, 13, 4807-4816 (2007) (Non-Patent Document 9); Mike, V. et al., International Journal of Cancer, 122, 2562-2567 (2008) (Non-Patent Document 10); and, Japanese Unexamined Patent Publication No. 2004-89180 (2008) (Patent Document 5)). This method consists of mixing two types of inner primers, two types of outer primers, reverse transcriptase, strand displacement DNA synthase and substrate, and amplifying a target nucleic acid by warming at a constant temperature (about 65°C). However, this method requires a large number of primers to be designed, and there are numerous restrictions on the design of each primer thereby making design extremely complex. For example, it is necessary to first select three domains each on the 3'-end and 5'-end for a target gene (and the distance between each domain is important at this time), and then design primers of different lengths by combining homologous or complementary sequences of each region. Since the Tm value, GC content and the like of each primer is related to reactivity at this time, those domains for which primers can be designed are limited considerably. Moreover, although it is possible to use a loop primer to increase the reaction rate (the use of a loop primer allows the detection time to be shortened to about 30 minutes), it is further necessary to design two primers for this purpose. Thus, not only is it difficult to design a large number of primers in this manner, but this method is also disadvantageous in terms of production cost. Moreover, in the LAMP method, amplification products are not detected directly, but rather the relative amounts of reaction products are estimated by detecting turbidity of a reaction solution. Since the method used to detect turbidity does not involve direct detection of amplified nucleic acids, not only can amplified nucleic acids not be detected quantitatively in the case non-specific amplification products are produced, but it is also not possible to determine whether or not non-specific amplification products have been produced based on the measurement results. In addition, since the LAMP method is characterized by a large number of primers being involved in the reaction, it is difficult to completely inhibit the production of non-specific amplification products. Thus, this method is not suited to simultaneous detection and amplification of multiple tumor markers as in Multiplex PCR as reported for some PCR methods. This is because the method used to detect turbidity prevents a determination from being made as to which tumor marker have been amplified and detected. In addition, since the LAMP method uses double-stranded DNA as a template in the same manner as RT-PCR, there is the potential for amplification of chromosomal DNA. In addition, although the temperature during amplification in the LAMP method is constant at 65°C, since high-temperature treatment is typically required before and after the reaction, this treatment is an obstacle to saving on energy and reducing costs of the reaction apparatus.

On the other hand, examples of other methods used to amplify only RNA at a constant temperature include NASBA (refer to Japanese Patent Publication No. 2650159 (Patent Document 6) and Japanese Patent Publication No. 3152927 (Patent Document 7) and TMA (Japanese Patent Publication No. 3241717 (Patent Document 8)). These RNA amplification methods involve synthesizing double-stranded DNA containing a promoter sequence by use of a primer specific to a target RNA wherein the primer comprises the promoter sequence, reverse transcriptase and, as necessary, ribonuclease H (RNase H), producing RNA containing a specific base sequence derived from the target RNA by use of RNA polymerase with using the double-stranded DNA as template, and using this RNA to carry out a chain reaction using double-stranded DNA containing a promoter sequence as template. Following RNA amplification, the amplified RNA is detected by electrophoresis or a hybridization method using a nucleic acid probe bound with a detectable label.

Although these RNA amplification methods are suitable for easily measuring RNA since they amplify only RNA at a constant temperature and in a single step, since the hybridization procedure and the like requires a complex procedure, not only are they not suitable for large-volume specimen processing and automation, they also have the shortcomings of poor reproducibility and the potential for secondary contamination by amplified nucleic acids as a result thereof. In addition, it normally takes 90 minutes or more to obtain results for both NASBA and TMA, thus preventing results from being obtained rapidly. Moreover, although the amplification step is carried out at a constant temperature, since the amplification step usually requires preheating (at a temperature of, for example, 65°C), these methods have shortcomings with respect to labor saving and reducing costs of the reaction apparatus.

A. C. Lambrechts et al., Breast and Cancer Research and Treatment 56: 219-231, 1999, discloses a method for detecting CK19, said method applying a standard NASBA method requiring a denaturation step before the RNA amplification reaction, wherein the detection limit of detecting carcinoma cells is 30 carcinoma cells in 6x10⁶ of normal cells.

An example of a method for easily amplifying and measuring RNA is the method of Ishiguro, et al. (refer to Japanese Unexamined Patent Publication No. 2000-14400 (Patent Document 9) and Ishiguro, T. et al., Analytical Biochemistry, 314, 1247-1252 (2003) (Non-Patent Document 11)). This method involves carrying out RNA amplification in the presence of an oligonucleotide probe labeled with an intercalating fluorescent dye and designed so that when it forms a complementary double-strand with the target nucleic acid, the intercalating fluorescent dye moiety undergoes a change in fluorescent properties due to intercalation into the double-stranded moiety, and measuring the change in fluorescent properties, thereby enabling amplification and measurement of RNA to be carried out simultaneously, rapidly and easily at a constant temperature, in a single step and in a closed vessel. In a more specific aspect thereof, this method consists of carrying out the following steps on a specific base sequence that allows an arbitrary RNA to be distinguished from other RNA in the presence of that RNA:
(1) forming a DNA complementary to the specific base sequence by use of a DNA primer complementary to the 3'-end of the specific base sequence, RNA-dependent DNA polymerase (reverse transcriptase) and RNA as a template,
(2) forming a single-stranded DNA by allowing an enzyme having ribonuclease H activity to act on the double-stranded RNA-DNA formed by the reverse transcription reaction of (1) to decompose the RNA,
(3) synthesizing a double-stranded DNA containing an RNA polymerase promoter sequence by use of a DNA primer complementary to the 3'-end of the single-stranded DNA formed in (2) and having the RNA polymerase promoter sequence on the 5'-end thereof, and DNA-dependent DNA polymerase, and
(4) forming a transcription product (the RNA of the specific base sequence) by allowing RNA polymerase to act on the double-stranded DNA formed in (3).

Since the RNA transcription product formed in (4) is an RNA derived from a specific base sequence, it serves as a template in the reaction of (1), binds to the DNA primer used in the reaction of (1), and allows the reactions of (1) to (4) to proceed to cause an RNA amplification chain reaction.

This nucleic acid amplification method is characterized by not requiring the temperature of the reaction solution to be raised and lowered in the manner of PCR, and carrying out reverse transcription of RNA and the subsequent DNA amplification reaction separately. Moreover, the state of amplification can be detected (monitored) simultaneous to amplifying the specific base sequence or sequence complementary to that sequence by also incorporating the oligonucleotide probe labeled with the intercalating fluorescent dye capable of specifically binding to the amplified RNA transcription product present in the reaction solution. Thus, since it is not necessary to carry out hybridization detection and the like separately in the manner of ordinary RNA amplification, the amount of time required for results to be obtained can be shortened considerably.

However, a primer sequence for amplification of CK19 mRNA suitable for this nucleic acid amplification method or combinations thereof, or an oligonucleotide probe sequence for detection, are currently not known. This is because, in comparison with nucleic acid amplification methods such as PCR, LAMP, NASBA or TMA, which require a step for temporarily raising the temperature at the start of the reaction to a temperature higher than the reaction temperature to denature the high-dimensional structure of the target RNA, amplification and detection of mRNA in this nucleic acid amplification method are carried out under constant, comparatively low temperature conditions (from 40 to 60°C, and preferably 43°C). In other words, typical single-stranded RNA in the manner of mRNA is known to easily form a high-dimensional structure, and under reaction conditions like those of this nucleic acid amplification method, the target mRNA forms a high-dimensional structure, and since this is thought to impair binding of the primer and probe, it is necessary to design an optimum primer and probe in a region that does not have a high-dimensional structure. Although it is possible to calculate and estimate secondary structure from nucleic acid sequences using secondary structure analytical software as an indicator of RNA high-dimensional structure, it is extremely difficult to estimate actual high-dimensional structure from a calculated secondary structure.

In addition, in the case of carrying out nucleic acid amplification at a comparatively low temperature as in this nucleic acid amplification method, non-specific products such as primer dimers form easily in comparison with PCR carried out at a high temperature, and in order to reduce the formation of non-specific products, it is necessary to carefully select the combination of primers used. However, since commonly used primer design techniques are premised on containing a step involving denaturation at a high temperature (e.g., PCR), it is difficult to design primers suitable for this nucleic acid amplification method using known primer design techniques. Accordingly, in order to realize highly sensitive measurement of CK19 mRNA both rapidly and easily by amplifying and measuring mRNA at a constant temperature as described above, an oligonucleotide and combinations thereof are required that do not demonstrate a decrease in binding efficiency and enable amplification and detection of CK19 mRNA even under constant temperature conditions (from 40 to 60°C, and preferably 43°C).

### SUMMARY OF THE INVENTION

An object of the present invention is to provide a method of rapidly measuring cytokeratin 19 mRNA with a procedure carried out at a constant temperature and in a single step on a sample obtained from human cells or tissue and the like.

Extensive studies to solve the aforementioned problems conducted by the inventors of the present invention led to the construction of a method of specifically and rapidly measuring cytokeratin (CK) mRNA.

The present disclosure concerns a method of measuring CK19 mRNA in a sample, consisting of the following steps that uses a first primer having a sequence homologous to a portion of a specific base sequence in CK19 mRNA, and a second primer having a sequence complementary to a portion of the specific base sequence, and one of either the first primer or the second primer is a primer to which has been added to the 5'-end thereof an RNA polymerase promoter sequence:
(1) a step for synthesizing a cDNA complementary to the specific base sequence by use of an enzyme having RNA-dependent DNA polymerase activity that uses RNA as a template;
(2) a step for decomposing the RNA-DNA double-stranded RNA obtained in the reaction of (1) above by use of an enzyme having ribonuclease H (RNase H) activity (formation of single-stranded DNA);
(3) a step for forming double-stranded DNA having a promoter sequence capable of transcribing the specific base sequence or RNA having a sequence complementary to the specific base sequence by use of an enzyme having DNA-dependent DNA polymerase activity with using the single-stranded DNA as template;
(4) a step for forming an RNA transcription product by use of an enzyme having RNA polymerase activity with using the double-stranded DNA as template;
(5) a step for forming an RNA transcription product in a chain reaction by allowing the RNA transcription product to serve as a template of cDNA synthesis in the reaction of (1); and,
(6) a step for measuring the amount of the RNA transcription product; wherein
   the first and second primers are any of the oligonucleotides indicated below:
   (i) the first primer is an oligonucleotide consisting of at least 15 contiguous bases in the base sequence listed as SEQ ID NO: 1, and the second primer is an oligonucleotide consisting of at least 15 contiguous bases in the base sequence listed as SEQ ID NO: 5;
   (ii) the first primer is an oligonucleotide consisting of at least 15 contiguous bases in the base sequence listed as SEQ ID NO: 2, and the second primer is an oligonucleotide consisting of at least 15 contiguous bases in the base sequence listed as SEQ ID NO: 6;
   (iii) the first primer is an oligonucleotide consisting of at least 15 contiguous bases in the base sequence listed as SEQ ID NO: 3, and the second primer is an oligonucleotide consisting of at least 15 contiguous bases in the base sequence listed as SEQ ID NO: 7; and,
   (iv) the first primer is an oligonucleotide consisting of at least 15 contiguous bases in the base sequence listed as SEQ ID NO: 4, and the second primer is an oligonucleotide consisting of at least 15 contiguous bases in the base sequence listed as SEQ ID NO: 8.

The first primer and the second primer can also be any of the oligonucleotides indicated below:
(i) the first primer is an oligonucleotide consisting of at least 15 contiguous bases in any of the base sequences listed as SEQ ID NO: 19 to 22, and the second primer is an oligonucleotide consisting of at least 15 contiguous bases in any of the base sequences listed as SEQ ID NO: 29 to 32;
(ii) the first primer is an oligonucleotide consisting of at least 15 contiguous bases in either of base sequence listed as SEQ ID NO: 23 or 24, and the second primer is an oligonucleotide consisting of at least 15 contiguous bases in either base sequence listed as SEQ ID NO: 33 or 34;
(iii) the first primer is an oligonucleotide consisting of at least 15 contiguous bases in either of base sequence listed as SEQ ID NO: 25 or 26, and the second primer is an oligonucleotide consisting of at least 15 contiguous bases in either base sequence listed as SEQ ID NO: 35 or 36; and,
(iv) the first primer is an oligonucleotide consisting of at least 15 contiguous bases in either of base sequence listed as SEQ ID NO: 27 or 28, and the second primer is an oligonucleotide consisting of at least 15 contiguous bases in either base sequence listed as SEQ ID NO: 37 or 38.
   Step (6) above (the step for measuring the amount of RNA transcription product) is carried out by measuring a change in fluorescent properties in the presence of a fluorescent dye-labeled oligonucleotide probe designed to change its fluorescent properties when it forms a complementary double-strand with the target RNA.

The fluorescent dye-labeled oligonucleotide probe is an intercalating fluorescent dye-labeled oligonucleotide probe in which an intercalating fluorescent dye is bound through a linker.

The intercalating fluorescent dye-labeled oligonucleotide probe contains an oligonucleotide consisting of at least 15 contiguous bases in any of the base sequences listed as SEQ ID NO: 39 to 46, or in a sequence complementary thereto.

Prior to the step described in (1) above (the step for synthesizing cDNA complementary to the specific base sequence by use of an enzyme having RNA-dependent DNA polymerase activity that uses RNA as a template), a step is carried out for cleaving the RNA at the 5'-end site of the specific base sequence by use of a specific base sequence in CK19 mRNA as a template, and using:
(i) a cleaving oligonucleotide having a region that overlaps with the 5'-end site of a region homologous to the first primer in the specific base sequence, and a sequence complementary to an adjacent region on the 5' side from the site, and
(ii) an enzyme having ribonuclease H (RNase H) activity.

The cleaving oligonucleotide is an oligonucleotide consisting of any of the base sequences listed as SEQ ID NO: 9 to 18.

### BRIEF DESCRIPTION OF THE DRAWINGS

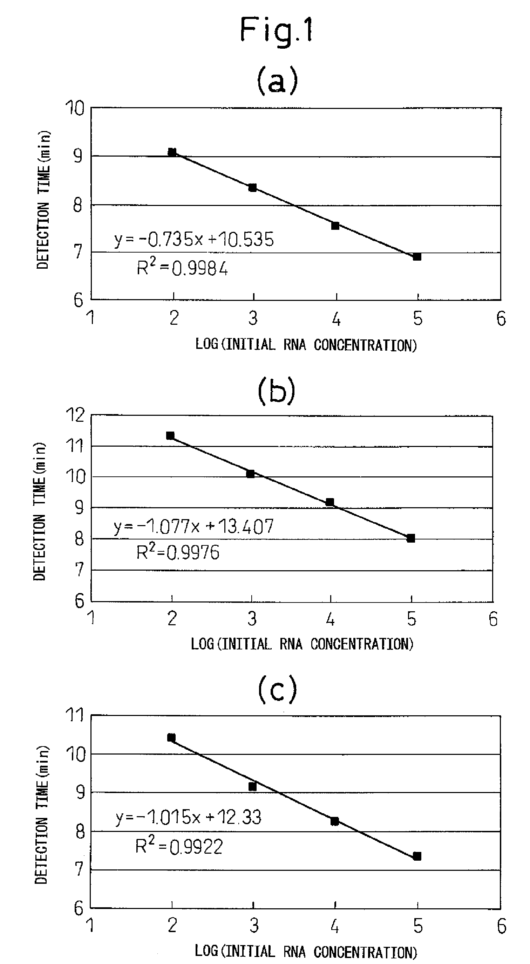

FIG. 1 is a calibration curve of CK19 RNA indicating an equation of a linear first order curve and a value of R² determined from each point, wherein (a) indicates oligonucleotide combination [8] of Table 1, (b) indicates combination [10] and (c) indicates combination [30], the time when the fluorescence intensity ratio has exceeded 1.2 (detection time, minutes) is plotted on the vertical axis, and the initial amount of standard RNA (number of copies) used for measurement represented as a log is plotted on the horizontal axis.

### DESCRIPTION OF THE PREFERRED EMBODIMENTS

The following provides a detailed explanation of the present invention.

The sample in the present invention refers to a nucleic acid sample that contains RNA. The present invention measures CK19 mRNA contained in human cells or tissue and the like serving as the source of the sample by using human cells, human tissue, body fluid, blood, urine, stool, lymph, nipple aspiration fluid or lavaged fluid from the peritoneal or thoracic cavity as a sample, using the sample prepared based on, for example, the method described in Japanese Unexamined Patent Publication No. H7-59572, and measuring the sample directly.

The specific base sequence in the present disclosure refers to an RNA or DNA base sequence of the CK19 mRNA starting from the 5'-end of a region homologous to the first primer and ending to the 3'-end of a region complementary to the second primer. Namely, in CK19 mRNA, the first primer is homologous to at least 15 contiguous bases in the 3' direction from the 5'-end of a specific base sequence, while the second primer is complementary to at least 15 contiguous bases in the 5' direction from the 3'-end of the specific bases sequence. Accordingly, in the present disclosure, an RNA transcription product is amplified that is derived from the specific base sequence. The 5'-end site of a region homologous to the first primer in the present disclosure refers to a site consisting of a partial sequence containing the 5'-end of the homologous region within the specific base sequence, and the site is a site where a region complementary to the cleaving oligonucleotide and a region homologous to the first primer overlap.

The promoter in the present invention refers to a site where transcription is initiated by binding by RNA polymerase. Although promoter sequences are known that are specific to various RNA polymerases and there are no particular limitations thereon, a promoter in the present invention is preferably a T7 promoter, SP6 promoter or T3 promoter that is normally used in molecular biology experiments and the like. In addition, an addition sequence involved in transcription efficiency may also be contained in the aforementioned sequence.

The complementary sequence in the present invention refers to a sequence that can hybridize with the target base sequence under highly stringent conditions. An example of highly stringent conditions is the composition of the nucleic acid amplification reaction solution described in the examples of the present invention. In addition, a homologous sequence in the present invention refers to a sequence that can hybridize with a completely complementary sequence of a target base sequence under highly stringent conditions. Thus, a complementary or homologous sequence as referred to in the present invention can naturally be set to an arbitrary length and the like provided it is within a range that does not affect specificity or efficiency of hybridization under highly stringent conditions. Moreover, a base sequence may be used in which one to a plurality of bases have been substituted, deleted or inserted within a range that does not affect specificity or efficiency of hybridization.

The nucleotide or nucleic acid in the present invention refers to a nucleotide or nucleoside (containing both RNA and DNA) consisting of bases, sugars and intersaccharide bonds that are present in nature, and is a generic term that includes oligomers thereof (oligonucleotides having, for example, about 2 to 100 bases) and polymers (oligonucleotides having, for example, 100 or more bases). A nucleotide or nucleic acid in the present invention includes similarly functioning monomers not present in nature, monomers labeled with a fluorescent molecule or radioisotope and the like, and oligomers or polymers in which they are contained.

The primer in the present invention refers to a nucleotide that hybridizes with a template in a nucleic acid amplification reaction and is required to initiate the nucleic acid amplification reaction, and in the nucleic acid amplification reaction, hybridizes with the template desired to be amplified, and is preferably designed on the base of the template to contain a sequence of the primer itself that is specific to the template so as to obtain a specific product in terms of strand length or sequence by a nucleic acid amplification reaction such as PCR, LAMP, ICAN, NASBA, TMA, 3SR or TRC (refer to Patent Document 9 and Non-Patent Document 11).

Although the primer is designed so as to have a strand length of normally 15 to 100 bases and preferably 15 to 35 bases, the primer length is not limited thereto. Accordingly, the first primer and the second primer of the present invention can be selected from arbitrary sequences of at least 15 contiguous bases within the range of base sequences described in the invention of the present application. Namely, the first primer and the second primer for detecting CK19 mRNA in the present disclosure are any of the oligonucleotides indicated below:
(i) the first primer is an oligonucleotide consisting of at least 15 contiguous bases in a sequence homologous to the sequence listed as SEQ ID NO: 1 that is homologous to a portion of CK19 mRNA, and the second primer is an oligonucleotide consisting of at least 15 contiguous bases in a sequence homologous to the sequence listed as SEQ ID NO: 5 that is complementary to a portion of CK19 mRNA;
(ii) the first primer is an oligonucleotide consisting of at least 15 contiguous bases in a sequence homologous to the sequence listed as SEQ ID NO: 2 that is homologous to a portion of CK19 mRNA, and the second primer is an oligonucleotide consisting of at least 15 contiguous bases in a sequence homologous to the sequence listed as SEQ ID NO: 6 that is complementary to a portion of CK19 mRNA;
(iii) the first primer is an oligonucleotide consisting of at least 15 contiguous bases in a sequence homologous to the sequence listed as SEQ ID NO: 3 that is homologous to a portion of CK19 mRNA, and the second primer is an oligonucleotide consisting of at least 15 contiguous bases in a sequence homologous to the sequence listed as SEQ ID NO: 7 that is complementary to a portion of CK19 mRNA; and,
(iv) the first primer is an oligonucleotide consisting of at least 15 contiguous bases in a sequence homologous to the sequence listed as SEQ ID NO: 4 that is homologous to a portion of CK19 mRNA, and the second primer is an oligonucleotide consisting of at least 15 contiguous bases in a sequence homologous to the sequence listed as SEQ ID NO: 8 that is complementary to a portion of CK19 mRNA.

In a more preferable aspect of the present disclosure the first primer and the second primer for detecting CK19 mRNA are any of the oligonucleotides indicated below:
(i) the first primer is an oligonucleotide consisting of at least 15 contiguous bases in a sequence homologous to any of the sequences listed as SEQ ID NO: 19 to 22 that are homologous to a portion of CK19 mRNA, and the second primer is an oligonucleotide consisting of at least 15 contiguous bases in a sequence homologous to any of the sequences listed as SEQ ID NO: 29 to 32 that are complementary to a portion of CK19 mRNA;
(ii) the first primer is an oligonucleotide consisting of at least 15 contiguous bases in a sequence homologous to either sequence listed as SEQ ID NO: 23 or 24 that is homologous to a portion of CK19 mRNA, and the second primer is an oligonucleotide consisting of at least 15 contiguous bases in a sequence homologous to either sequence listed as SEQ ID NO: 33 or 34 that is complementary to a portion of CK19 mRNA;
(iii) the first primer is an oligonucleotide consisting of at least 15 contiguous bases in a sequence homologous to either sequence listed as SEQ ID NO: 25 or 26 that is homologous to a portion of CK19 mRNA, and the second primer is an oligonucleotide consisting of at least 15 contiguous bases in a sequence homologous to either sequence listed as SEQ ID NO: 35 or 36 that is complementary to a portion of CK19 mRNA; and,
(iv) the first primer is an oligonucleotide consisting of at least 15 contiguous bases in a sequence homologous to either sequence listed as SEQ ID NO: 27 or 28 that is homologous to a portion of CK19 mRNA, and the second primer is an oligonucleotide consisting of at least 15 contiguous bases in a sequence homologous to either sequence listed as SEQ ID NO: 37 or 38 that is complementary to a portion of CK19 mRNA.

Examples of preferable aspects of the first primer and the second primer for detecting CK19 mRNA in the present disclosure include any of the oligonucleotides indicated below:
(i) the first primer is one type of oligonucleotide selected from sequences listed as SEQ ID NO: 19 to 22, and the second primer is one type of oligonucleotide selected from sequences listed as SEQ ID NO: 29 to 32;
(ii) the first primer is one type of oligonucleotide selected from a sequence listed as SEQ ID NO: 23 or 24, and the second primer is one type of oligonucleotide selected from a sequence listed as SEQ ID NO: 33 or 34;
(iii) the first primer is one type of oligonucleotide selected from a sequence listed as SEQ ID NO: 25 or 26, and the second primer is one type of oligonucleotide selected from a sequence listed as SEQ ID NO: 35 or 36; and,
(iv) the first primer is one type of oligonucleotide selected from a sequence listed as SEQ ID NO: 27 or 28, and the second primer is one type of oligonucleotide selected from a sequence listed as SEQ ID NO: 37 or 38.

Moreover, in one aspect of the present disclosure, CK19 mRNA is cleaved at the 5'-end site of a specific nucleic acid sequence within the RNA prior to serving as a template of cDNA synthesis. As a result of being cleaved at the 5'-end site of a specific nucleic acid sequence, a DNA strand complementary to the promoter sequence of the first primer hybridized to cDNA can be efficiently synthesized following cDNA synthesis by elongating the 3'-end of the cDNA, thereby enabling the formation of a functional double-stranded DNA promoter structure. An example of such a cleavage method consists of cleaving an RNA portion of double-stranded RNA-DNA, formed by adding an oligonucleotide having a sequence complementary to a region that overlaps with a 5'-end site of the specific base sequence within CK19 mRNA (partial sequence containing the 5'-end site of the specific base sequence) and is adjacent thereto in the 5'-direction (to be referred to as a cleaving oligonucleotide), with an enzyme having ribonuclease (RNase H) activity. The hydroxyl group on the 3'-end of the cleaving oligonucleotide is preferably suitably modified to prevent an elongation reaction, an example of which is an aminated hydroxyl group.

In a preferable aspect of the present disclosure the cleaving oligonucleotide is an oligonucleotide consisting of a sequence homologous to any of the sequences listed as SEQ ID NO: 9 to 18 that are complementary to a portion of CK19 mRNA.

The target RNA in the present disclosure refers to a sequence among specific base sequences of an RNA transcription product other than a region that is homologous or complementary to the aforementioned primers, and has a sequence capable of complementarily binding with an intercalating fluorescent dye-labeled oligonucleotide probe. Accordingly, the intercalating fluorescent dye-labeled oligonucleotide probe is a sequence that is complementary or homologous to a portion of the specific base sequence in the present invention. In one aspect of the present disclosure an example of the intercalating fluorescent dye-labeled oligonucleotide probe is a probe containing an oligonucleotide consisting of at least 15 contiguous bases in a sequence homologous or complementary to SEQ ID NO: 39 to 46 that are complementary to a portion of CK19 mRNA.

In one aspect of a combination of oligonucleotides for detecting CK19 mRNA in the present disclosure:
(i) the cleaving oligonucleotide is an oligonucleotide consisting of a sequence homologous to any of the sequences listed as SEQ ID NO: 9 to 12, the first primer is an oligonucleotide consisting of at least 15 contiguous bases in a sequence homologous to the sequence listed as SEQ ID NO: 1 (and furthermore, the first primer has a promoter sequence on the 5'-end thereof, and a 5'-end site of a region homologous to the primer in a specific nucleic acid sequence overlaps with a complementary region of a sequence listed as SEQ ID NO: 9 to 12), the second primer is an oligonucleotide of at least 15 contiguous bases in a sequence homologous to the sequence listed as SEQ ID NO: 5, and the intercalating fluorescent dye-labeled oligonucleotide probe is a probe containing an oligonucleotide consisting of at least 15 contiguous bases in a sequence homologous to any of the sequences listed as SEQ ID NO: 39 to 41;
(ii) the cleaving oligonucleotide is an oligonucleotide consisting of a sequence homologous to a sequence listed as SEQ ID NO: 13 or 14, the first primer is an oligonucleotide consisting of at least 15 contiguous bases in a sequence homologous to the sequence listed as SEQ ID NO: 2 (and furthermore, the first primer has a promoter sequence on the 5'-end thereof, and a 5'-end site of a region that is homologous to the primer in a specific nucleic acid sequence overlaps with a complementary region of a sequence listed as SEQ ID NO: 13 or 14), the second primer is an oligonucleotide consisting of at least 15 contiguous bases in a sequence homologous to the sequence listed as SEQ ID NO: 6, and the intercalating fluorescent dye-labeled oligonucleotide probe is a probe containing an oligonucleotide consisting of at least 15 contiguous bases in a sequence homologous to a sequence listed as SEQ ID NO: 42 or 43;
(iii) the cleaving oligonucleotide is an oligonucleotide consisting of a sequence homologous to a sequence listed as SEQ ID NO: 15 or 16, the first primer is an oligonucleotide consisting of at least 15 contiguous bases in a sequence homologous to the sequence listed as SEQ ID NO: 3 (and furthermore, the first primer has a promoter sequence on the 5'-end thereof, and a 5'-end site of a region that is homologous to the primer in a specific nucleic acid sequence overlaps with a complementary region of a sequence listed as SEQ ID NO: 15 or 16), the second primer is an oligonucleotide consisting of at least 15 contiguous bases in a sequence homologous to the sequence listed as SEQ ID NO: 7, and the intercalating fluorescent dye-labeled oligonucleotide probe is a probe containing an oligonucleotide consisting of at least 15 contiguous bases in a sequence homologous to a sequence listed as SEQ ID NO: 44 or 45; and,
(iv) the cleaving oligonucleotide is an oligonucleotide consisting of a sequence homologous to a sequence listed as SEQ ID NO: 17 or 18, the first primer is an oligonucleotide consisting of at least 15 contiguous bases in a sequence homologous to the sequence listed as SEQ ID NO: 4 (and furthermore, the first primer has a promoter sequence on the 5'-end thereof, and a 5'-end site of a region that is homologous to the primer in a specific nucleic acid sequence overlaps with a complementary region of a sequence listed as SEQ ID NO: 17 or 18), the second primer is an oligonucleotide consisting of at least 15 contiguous bases in a sequence homologous to the sequence listed as SEQ ID NO: 8, and the intercalating fluorescent dye-labeled oligonucleotide probe is a probe containing an oligonucleotide consisting of at least 15 contiguous bases in a sequence homologous to the sequence listed as SEQ ID NO: 46. Furthermore, in the case of (i), it is necessary to design each oligonucleotide so that a complementary sequence of the intercalating fluorescent dye-labeled oligonucleotide probe does not overlap with the homologous sequence of the first primer and the complementary sequence of the second primer.

Examples of more preferable aspects of combinations of oligonucleotides for detecting CK19 mRNA in the present disclosure include the following:
(i) the cleaving oligonucleotide is an oligonucleotide consisting of the sequence listed as SEQ ID NO: 9, the first primer is an oligonucleotide consisting of the sequence listed as SEQ ID NO: 19 (and furthermore, the first primer has a promoter sequence on the 5'-end thereof), the second primer is one type of oligonucleotide selected from a sequence listed as SEQ ID NO: 29 or 30, and the intercalating fluorescent dye-labeled oligonucleotide probe is one type of oligonucleotide selected from a sequence listed as SEQ ID NO: 39 or 40;
(ii) the cleaving oligonucleotide is an oligonucleotide consisting of the sequence listed as SEQ ID NO: 9, the first primer is an oligonucleotide consisting of the sequence listed as SEQ ID NO: 19 (and furthermore, the first primer has a promoter sequence on the 5'-end thereof), the second primer is one type of oligonucleotide selected from a sequence listed as SEQ ID NO: 31 or 32, and the intercalating fluorescent dye-labeled oligonucleotide probe is one type of oligonucleotide selected from a sequence listed as SEQ ID NO: 39 to 41;
(iii) the cleaving oligonucleotide is an oligonucleotide consisting of the sequence listed as SEQ ID NO: 10, the first primer is an oligonucleotide consisting of the sequence listed as SEQ ID NO: 20 (and furthermore, the first primer has a promoter sequence on the 5'-end thereof), the second primer is one type of oligonucleotide selected from a sequence listed as SEQ ID NO: 29 or 30, and the intercalating fluorescent dye-labeled oligonucleotide probe is one type of oligonucleotide selected from a sequence listed as SEQ ID NO: 39 or 40;
(iv) the cleaving oligonucleotide is an oligonucleotide consisting of the sequence listed as SEQ ID NO: 10, the first primer is an oligonucleotide consisting of the sequence listed as SEQ ID NO: 20 (and furthermore, the first primer has a promoter sequence on the 5'-end thereof), the second primer is one type of oligonucleotide selected from a sequence listed as SEQ ID NO: 31 or 32, and the intercalating fluorescent dye-labeled oligonucleotide probe is one type of oligonucleotide selected from a sequence listed as SEQ ID NO: 39 to 41;
(v) the cleaving oligonucleotide is an oligonucleotide consisting of the sequence listed as SEQ ID NO: 11, the first primer is an oligonucleotide consisting of the sequence listed as SEQ ID NO: 21 (and furthermore, the first primer has a promoter sequence on the 5'-end thereof), the second primer is one type of oligonucleotide selected from a sequence listed as SEQ ID NO: 29 or 30, and the intercalating fluorescent dye-labeled oligonucleotide probe is an oligonucleotide consisting of the sequence listed as SEQ ID NO: 40;
(vi) the cleaving oligonucleotide is an oligonucleotide consisting of the sequence listed as SEQ ID NO: 11, the first primer is an oligonucleotide consisting of the sequence listed as SEQ ID NO: 21 (and furthermore, the first primer has a promoter sequence on the 5'-end thereof), the second primer is one type of oligonucleotide selected from a sequence listed as SEQ ID NO: 31 or 32, and the intercalating fluorescent dye-labeled oligonucleotide probe is one type of oligonucleotide selected from a sequence listed as SEQ ID NO: 40 or 41;
(vii) the cleaving oligonucleotide is an oligonucleotide consisting of the sequence listed as SEQ ID NO: 12, the first primer is an oligonucleotide consisting of the sequence listed as SEQ ID NO: 22 (and furthermore, the first primer has a promoter sequence on the 5'-end thereof), the second primer is one type of oligonucleotide selected from a sequence listed as SEQ ID NO: 29 or 30, and the intercalating fluorescent dye-labeled oligonucleotide probe is an oligonucleotide consisting of the sequence listed as SEQ ID NO: 40;
(viii) the cleaving oligonucleotide is an oligonucleotide consisting of the sequence listed as SEQ ID NO: 12, the first primer is an oligonucleotide consisting of the sequence listed as SEQ ID NO: 22 (and furthermore, the first primer has a promoter sequence on the 5'-end thereof), the second primer is one type of oligonucleotide selected from a sequence listed as SEQ ID NO: 31 or 32, and the intercalating fluorescent dye-labeled oligonucleotide probe is one type of oligonucleotide selected from a sequence listed as SEQ ID NO: 40 or 41;
(ix) the cleaving oligonucleotide is an oligonucleotide consisting of the sequence listed as SEQ ID NO: 13, the first primer is an oligonucleotide consisting of the sequence listed as SEQ ID NO: 23 (and furthermore, the first primer has a promoter sequence on the 5'-end thereof), the second primer is one type of oligonucleotide selected from a sequence listed as SEQ ID NO: 33 or 34, and the intercalating fluorescent dye-labeled oligonucleotide probe is one type of oligonucleotide selected from a sequence listed as SEQ ID NO: 42 or 43;
(x) the cleaving oligonucleotide is an oligonucleotide consisting of the sequence listed as SEQ ID NO: 14, the first primer is an oligonucleotide consisting of the sequence listed as SEQ ID NO: 24 (and furthermore, the first primer has a promoter sequence on the 5'-end thereof), the second primer is one type of oligonucleotide selected from a sequence listed as SEQ ID NO: 33 or 34, and the intercalating fluorescent dye-labeled oligonucleotide probe is one type of oligonucleotide selected from a sequence listed as SEQ ID NO: 42 or 43;
(xi) the cleaving oligonucleotide is an oligonucleotide consisting of the sequence listed as SEQ ID NO: 15, the first primer is an oligonucleotide consisting of the sequence listed as SEQ ID NO: 25 (and furthermore, the first primer has a promoter sequence on the 5'-end thereof), the second primer is one type of oligonucleotide selected from a sequence listed as SEQ ID NO: 35 or 36, and the intercalating fluorescent dye-labeled oligonucleotide probe is one type of oligonucleotide selected from a sequence listed as SEQ ID NO: 44 or 45;
(xii) the cleaving nucleotide is an oligonucleotide consisting of the sequence listed as SEQ ID NO: 16, the first primer is an oligonucleotide consisting of the sequence listed as SEQ ID NO: 26 (and furthermore, the first primer has a promoter sequence on the 5'-end thereof), the second primer is one type of oligonucleotide selected from a sequence listed as SEQ ID NO: 35 or 36, and the intercalating fluorescent dye-labeled oligonucleotide probe is one type of oligonucleotide selected from a sequence listed as SEQ ID NO: 44 or 45;
(xiii) the cleaving oligonucleotide is an oligonucleotide consisting of the sequence listed as SEQ ID NO: 17, the first primer is an oligonucleotide consisting of the sequence listed as SEQ ID NO: 27 (and furthermore, the first primer has a promoter sequence on the 5'-end thereof), the second primer is one type of oligonucleotide selected from a sequence listed as SEQ ID NO: 37 or 38, and the intercalating fluorescent dye-labeled oligonucleotide probe is an oligonucleotide consisting of the sequence listed as SEQ ID NO: 46; and,
(xiv) the cleaving oligonucleotide is an oligonucleotide consisting of the sequence listed as SEQ ID NO: 18, the first primer is an oligonucleotide consisting of the sequence listed as SEQ ID NO: 28 (and furthermore, the first primer has a promoter sequence on the 5'-end thereof), the second primer is one type of oligonucleotide selected from a sequence listed as SEQ ID NO: 37 or 38, and the intercalating fluorescent dye-labeled oligonucleotide probe is an oligonucleotide consisting of the sequence listed as SEQ ID NO: 46.

Each enzyme is required in the method of measuring CK19 mRNA of the present invention (including an enzyme having RNA-dependent DNA polymerase activity using single-stranded RNA as a template (reverse transcriptase), an enzyme having RNase H activity, an enzyme having DNA-dependent DNA polymerase activity that uses single-stranded DNA as a template, and an enzyme having RNA polymerase activity). An enzyme having a combination of several activities may be used, or a plurality of enzymes having each activity may be used for each of the enzymes. In addition, not only may an enzyme having RNA polymerase activity be added to reverse transcriptase having three kinds of activities of RNA-dependent DNA polymerase activity which uses single-stranded RNA as a template, RNase H activity and DNA-dependent DNA polymerase activity which uses single-stranded DNA as a template, but also an enzyme having RNase H activity may be added as necessary. AMV reverse transcriptase commonly used in molecular biology experiments and the like, MMLV reverse transcriptase, HIV reverse transcriptase or derivatives thereof are preferable for the aforementioned reverse transcriptase, while AMV reverse transcriptase and derivatives thereof are the most preferable. In addition, examples of the aforementioned enzymes having RNA polymerase activity include bacteriophage-derived T7 RNA polymerase commonly used in molecular biology experiments and the like, T3 RNA polymerase, SP6 RNA polymerase and derivatives thereof.

In one aspect of the present invention, the cleaving oligonucleotide is added to CK19 mRNA in a sample, and the RNA is cleaved at a 5'-end site of the specific base sequence by RNase H activity of the aforementioned reverse transcriptase. When a reverse transcription reaction is carried out with the reverse transcriptase in the presence of the first primer and the second primer by using the cleaved RNA as a template, the second primer binds to the specific base sequence within the CK19 mRNA, and cDNA synthesis is carried out by RNA-dependent DNA polymerase activity of the reverse transcriptase. The first primer binds to the cDNA as a result of the RNA moiety of the resulting double-stranded RNA-DNA being decomposed and dissociated by the RNase H activity of the reverse transcriptase. Continuing, double-stranded DNA derived from the specific base sequence and having a promoter sequence on the 5'-end thereof is formed by the DNA-dependent DNA polymerase activity of the reverse transcriptase. This double-stranded DNA contains the specific base sequence downstream from the promoter sequence, and produces an RNA transcription product derived from the specific base sequence by the RNA polymerase. The RNA transcription product serves as a template for the double-stranded DNA synthesis by the first and second primers, and the RNA transcription product is amplified as a result of a series of reactions proceeding in the manner of a chain reaction.

In order to allow this chain reaction to proceed, it goes without saying that each of the enzymes at least contains as essential known elements a buffer, a magnesium salt, a potassium salt, a nucleoside triphosphate and a ribonucleoside triphosphate. In addition, dimethylsulfoxide (DMSO), dithiothreitol (DTT), bovine serum albumin (BSA) and a sugar and the like may also be added as additives for adjusting reaction efficiency.

For example, in the case of using AMV reverse transcriptase and T7 RNA polymerase, the reaction temperature is preferably set within the range of 35 to 65°C, more preferably within the range of 40 to 60°C, and even more preferably to 43°C. The reaction temperature can be set to an arbitrary temperature at which the RNA amplification step proceeds at a constant temperature and the reverse transcriptase and RNA polymerase demonstrate activity.

The amplified RNA transcription product can be measured according to a known nucleic acid measurement method. Examples of such methods include a method that uses electrophoresis or liquid chromatography, and a hybridization method that uses a nucleic acid probe labeled with a detectable label. However, the procedures of these methods have a large number of steps, and there is a considerable risk of dispersion of the amplification product into the atmosphere causing secondary contamination since the amplification product is analyzed by removing from the reaction system. In order to overcome these shortcomings, it is preferable to use an oligonucleotide probe designed so that fluorescent properties change due to complementary binding with a target nucleic acid. Although a known probe that uses FRET in the manner of a molecular beacon can be used for the oligonucleotide probe, in consideration of ease of designing the probe and ease of probe synthesis, a more preferable method consists of carrying out the nucleic acid amplification step in the presence of an oligonucleotide probe, which is labeled with an intercalating fluorescent dye and designed so that when it forms a complementary double strand with the target nucleic acid, fluorescent properties thereof change as a result of the intercalating fluorescent dye moiety intercalating into the complementary double-strand moiety followed by measuring the change in fluorescent properties (see Patent Document 9 and Non-Patent Document 11).

Although there are no particular limitations thereon, examples of the intercalating fluorescent dye that can be used include commonly used oxazole yellow, thiazole orange, ethidium bromide and derivatives thereof. An example of the change in fluorescent properties described above is a change in fluorescence intensity. In the case of oxazole yellow, for example, fluorescence at 510 nm (excitation wavelength: 490 nm) is known to increase remarkably following intercalation into double-stranded DNA. The aforementioned intercalating fluorescent dye-labeled oligonucleotide probe has a structure in which an intercalating fluorescent dye is bound to an end, phosphate diester moiety or nucleotide moiety in an oligonucleotide complementary to a target RNA of the RNA transcription product through a suitable linker, and the hydroxyl group on the 3'-end is suitably modified for the purpose of preventing elongation from the hydroxyl group on the 3'-end (see Patent Document 9 and Non-Patent Document 11).

Labeling of the intercalating fluorescent dye to an oligonucleotide can be carried out by binding the intercalating fluorescent dye by introducing a functional group into an oligonucleotide using a known method (see Patent Document 9 and Non-Patent Document 11). In addition, commercially available Label-ON Reagents (Clontech) and the like can also be used to introduce the functional group.

In one aspect of the present disclosure, a method is provided consisting of adding an amplification reagent, at least containing a first primer having a T7 promoter sequence (SEQ ID NO: 47) on the 5'-end thereof, a second primer, an intercalating fluorescent dye-labeled oligonucleotide probe, a cleaving oligonucleotide, AMV reverse transcriptase, T7 RNA polymerase, buffer, magnesium salt, potassium salt, nucleoside triphosphate, ribonucleoside triphosphate and dimethylsulfoxide (DMSO), to a sample, and then reacting at a constant reaction temperature of 40 to 60°C (and preferably 43°C) simultaneous to measuring fluorescence intensity of the reaction solution over time.

In this aspect, since fluorescence intensity is measured over time, measurement can be completed at an arbitrary time at which a significant increase in fluorescence has been observed, and can usually be completed within 30 minutes for both nucleic acid amplification and measurement.

In one aspect of the present invention, the amount of the specific base sequence (number of copies of a target RNA) present in a sample can be calculated by measuring CK19 mRNA in the sample and comparing resulting information on fluorescence intensity ratio with information on fluorescence intensity ratio when CK19 mRNA was measured having a known concentration. Although a sandwich assay can be applied to detect the specific base sequence by using an immobilized and labeled probe capable of complementarily binding to the specific base sequence in a reaction solution in which the aforementioned reaction was carried out for a fixed period of time, as was previously described, a method that uses an intercalating fluorescent dye-labeled oligonucleotide probe that specifically binds to the specific base sequence is preferable. In addition, since this probe does not impair the aforementioned RNA amplification reaction, a method consisting of carrying out amplification of the specific nucleic acid sequence in the presence of this probe and monitoring the specific nucleic acid sequence amplification status is particularly preferable. Furthermore, in the case of carrying out amplification of a specific nucleic acid sequence in the presence of an intercalating fluorescent dye-labeled oligonucleotide probe, it is preferable to add glycolic acid or biotin to the 3'-end thereof, for example, so as to prevent the probe moiety from functioning as an elongation reaction primer. By then measuring a fluorescence signal emitted as a result of the intercalating fluorescent dye-labeled oligonucleotide probe binding to the specific nucleic acid sequence during the amplification reaction with a fluorescence detector, and comparing information obtained from a profile thereof (for example, reaction time required for intensity of fluorescence emitted by the fluorescent dye to reach a fixed intensity) with information obtained from a profile relating to a known amount of standard RNA, the presence or absence of the specific nucleic acid sequence can be confirmed, or the amount of the specific nucleic acid sequence present in the sample (number of copies of a target RNA) can be estimated from the amount of specific nucleic acid sequence (number of RNA copies) that has been amplified.

In addition, it is noteworthy that all samples contained in the measurement reagent can be sealed within a single container. Namely, by simply carrying out a procedure in which a fixed amount of a sample is dispensed into that single container, CK19 mRNA can then be subsequently measured automatically. As long as at least a portion of the container is consisting of a transparent material so as to enable a signal emitted by the fluorescent dye to be measured from the outside, for example, a container that can be sealed after dispensing the sample is particularly preferable in terms of preventing contamination.

Since the RNA amplification and measurement method of the previous aspects can be carried out in a single step and at a constant temperature, it can be said to be simpler and more suitable to automation than RT-PCR. The present invention has made it possible to amplify and detect CK19 mRNA with high specificity, high sensitivity, rapidly, easily and at a constant temperature and in a single step.

In the present disclosure an initial amount of RNA can be determined both easily and rapidly by analyzing the course of increases in fluorescence intensity in a step in which double-stranded DNA having a DNA-dependent RNA polymerase promoter region on the 5'-end thereof is synthesized based on a target RNA in a sample (mRNA of CK19 gene), a large amount of single-stranded RNA is formed by using this DNA as a template to dramatically increase the amount of the single-stranded RNA formed, and an increase in fluorescence is measured by allowing an oligonucleotide probe labeled with an intercalating fluorescent dye to complementarily bind with the single-stranded RNA formed. The combined nucleic acid amplification and measurement time of the CK19 mRNA measurement method of the present invention is 30 minutes or less, and this is faster than measurement using a conventional method such as RT-PCR (normally 2 hours or more), NASBA (90 minutes or more), TMA (90 minutes or more) or RT-LAMP (normally from about 30 to 60 minutes).

Moreover, by providing an oligonucleotide for amplifying and detecting mRNA of CK19 gene in a single step, namely by providing an oligonucleotide for amplifying CK19 mRNA and an oligonucleotide for detecting CK19 mRNA, a simple, fast and highly sensitive method for measuring cells expressing CK19 mRNA, and a quantification reagent for use in the fields of biochemistry, molecular biology and medicine, can be provided by using this oligonucleotide.

### Examples

Although the following provides a more detailed explanation of the present invention through examples thereof, these examples are only intended to be exemplary, and the present invention is not limited by these examples.

### Example 1 Preparation of Reference RNA

CK19 RNA used in the following examples (to be referred to as standard RNA) was prepared using the methods indicated (1) to (2) below.
(1) Double-stranded DNA was cloned consisting of nucleotides from positions 160 to 1353 (1194 bases) of a CK19 base sequence registered with GenBank (GenBank Accession No. NM_002276, 1490 bases).
(2) In vitro transcription was carried out using the double-stranded DNA prepared in (1) as a template. Continuing, RNA was prepared after completely digesting the double-stranded DNA by treating with DNase I and purifying. The RNA was quantified by measuring absorbance at 260 nm.

### Example 2 Preparation of Intercalating Fluorescent

### Dye-Labeled Oligonucleotide Probe

An oligonucleotide probe was prepared that was labeled with an intercalating fluorescent dye. Amino groups were introduced using Label-ON Reagents (Clontech) at the location of the 9th T from the 5'-end of the sequence listed as SEQ ID NO: 39, the 12th T from the 5'-end of the sequence listed as SEQ ID NO: 40, the 13th A from the 5'-end of the sequence described in SEQ ID NO: 41, the 13th C from the 5'-end of the sequence listed as SEQ ID NO: 42, the 7th T from the 5'-end of the sequence listed as SEQ ID NO: 43, the 11th G from the 5'-end of the sequence listed as SEQ ID NO: 44, the 10th C from the 5'-end of the sequence listed as SEQ ID NO: 45, and the 9th G from the 5'-end of the sequence listed as SEQ ID NO: 46, followed by further modifying the 3'-end with biotin. Oxazole yellow serving as intercalating fluorescent dye was labeled to the amino groups to prepare oxazole yellow-labeled oligonucleotide probes (SEQ ID NO: 39 to 46) (see Ishiguro, T. et al., Nucleic Acids Res., 24, 4992-4997 (1996) (Non-Patent Document 12)).

### Example 3 Measurement of CK19 RNA (Part 1)

Standard RNA was measured according to the method indicated in (1) to (4) using the cleaving oligonucleotides, first primers, second primers and intercalating fluorescent dye-labeled oligonucleotide probes (to be referred to as "INAF probes") indicated in the combinations of [1] to [28] shown in Table 1. Furthermore, in Table 1, SEQ ID NO: 19 to 22 constitute partial sequences of SEQ ID NO: 1, SEQ ID NO: 23 and 24 constitute partial sequences of SEQ ID NO: 2, SEQ ID NO: 25 and 26 constitute partial sequences of SEQ ID NO: 3, SEQ ID NO: 27 and 28 constitute partial sequences of SEQ ID NO: 4, SEQ ID NO: 29 to 32 constitute partial sequences of SEQ ID NO: 5, SEQ ID NO: 33 and 34 constitute partial sequences of SEQ ID NO: 6, SEQ ID NO: 35 and 36 constitute partial sequences of SEQ ID NO: 7, and SEQ ID NO: 37 and 38 constitute partial sequences of SEQ ID NO: 8.

**Table 1**

| Oligonucleotide combination | Oligonucleotides Used (SEQ ID NO) | | | |
|---|---|---|---|---|
| | Cleaving oligonucleotide | First primer | Second primer | INAF probe |
| [1] | 9 | 19 | 29 | 39 |
| [2] | 9 | 19 | 30 | 39 |
| [3] | 10 | 20 | 29 | 39 |
| [4] | 10 | 20 | 30 | 39 |
| [5] | 11 | 21 | 31 | 41 |
| [6] | 11 | 21 | 32 | 41 |
| [7] | 12 | 22 | 31 | 41 |
| [8] | 12 | 22 | 32 | 41 |
| [9] | 13 | 23 | 33 | 42 |
| [10] | 13 | 23 | 33 | 43 |
| [11] | 13 | 23 | 34 | 42 |
| [12] | 13 | 23 | 34 | 43 |
| [13] | 14 | 24 | 33 | 42 |
| [14] | 14 | 24 | 33 | 43 |
| [15] | 14 | 24 | 34 | 42 |
| [16] | 14 | 24 | 34 | 43 |
| [17] | 15 | 25 | 35 | 44 |
| [18] | 15 | 25 | 35 | 45 |
| [19] | 15 | 25 | 36 | 44 |
| [20] | 15 | 25 | 36 | 45 |
| [21] | 16 | 26 | 35 | 44 |
| [22] | 16 | 26 | 35 | 45 |
| [23] | 16 | 26 | 36 | 44 |
| [24] | 16 | 26 | 36 | 45 |
| [25] | 17 | 27 | 37 | 46 |
| [26] | 17 | 27 | 38 | 46 |
| [27] | 18 | 28 | 37 | 46 |
| [28] | 18 | 28 | 38 | 46 |
| [29] | 11 | 21 | 32 | 40 |
| [30] | 12 | 22 | 32 | 40 |

(1) The standard RNA prepared in Example 1 were diluted using an RNA diluent (10 mM Tris-HCl buffer (pH 8.0), 0.1 mM EDTA, 0.5 U/µL ribonuclease inhibitor, 5.0 mM DTT) to 10³ copies/5 µL and used as RNA samples.
(2) 20 µL aliquots of the reaction solution having the composition indicated below were dispensed into 0.5 mL volume PCR tubes (Individual PCR Tubes with Dome Cap, SSI) followed by the addition of 5 µL of the RNA samples thereto.
   Reaction Solution Composition (Final concentration after addition of enzyme solution (in 30 µL)) 60 mM Tris-HCl buffer (pH 8.6)
   18 mM magnesium chloride
   100 mM potassium chloride
   1.0 mM DTT
   0.25 mM each dATP, dCTP, dGTP, dTTP
   3.0 mM each ATP, CTP, UTP, GTP
   3.6 mM ITP
   1.0 µM first primer (a T7 promoter sequence (SEQ ID NO:47) was added to the 5'-end of the primer having the base sequence described in each SEQ ID NO)
   1.0 µM second primer
   0.16 µM cleaving oligonucleotide (a hydroxyl group on the 3'-end of the oligonucleotide is modified with an amino group)
   20 nM INAF probe
   6.0 U ribonuclease inhibitor (Takara Bio) 13% DMSO
   Distilled water for adjusting volume
(3) The reaction solution was warmed for 5 minutes at 43°C followed by adding 5.0 µL of an enzyme solution having the composition indicated below pre-warmed for 2 minutes at 43°C.
   Enzyme Solution Composition (Final concentration at time of reaction (in 30 µL))
   2.0% Sorbitol
   6.4 U AMV reverse transcriptase (Life Science)
   142 U T7 RNA polymerase (Invitrogen)
   3.6 µg bovine serum albumin (Takara Bio)
   Distilled water for adjusting volume
(4) Continuing, fluorescence intensity of the reaction solution was measured (excitation wavelength: 470 nm, fluorescence wavelength: 510 nm) over time for 30 minutes simultaneous to reacting at 43°C using a fluorescence spectrophotometer equipped with a temperature control function capable of measuring the PCR tubes directly.

Designating the time of addition of enzyme as 0 minutes, samples were judged to be positive when the fluorescence intensity ratio of the reaction solution (fluorescence intensity value at a prescribed time ÷ background fluorescence intensity value) exceeded 1.2, and results showing the elapsed times at that time are shown in Table 2 as detection times.

**Table 2**

| Oligonucleotide combination | Detection time (min) (10³ copies/test) | Oligonucleotide combination | Detection time (min) (10³ copies/test) |
|---|---|---|---|
| [1] | 15.62 | [15] | 12.01 |
| [2] | 15.40 | [16] | 10.56 |
| [3] | 13.49 | [17] | 10.96 |
| [4] | 15.83 | [18] | 14.30 |
| [5] | 12.91 | [19] | 11.23 |
| [6] | 9.29 | [20] | 11.84 |
| [7] | 9.46 | [21] | 9.12 |
| [8] | 8.01 | [22] | 9.85 |
| [9] | 10.68 | [23] | 9.75 |
| [10] | 9.29 | [24] | 10.24 |
| [11] | 11.54 | [25] | 14.03 |
| [12] | 10.15 | [26] | 12.39 |
| [13] | 10.74 | [27] | 10.03 |
| [14] | 9.68 | [28] | 10.04 |

Among the combinations of Table 1:
(i) in those combinations in which the first primer is consisting of a partial sequence of SEQ ID NO: 1 and the second primer is consisting of a partial sequence of SEQ ID NO: 5 (combinations [1] to [8]),
(ii) in those combinations in which the first primer is consisting of a partial sequence of SEQ ID NO: 2 and the second primer is consisting of a partial sequence of SEQ ID NO: 6 (combinations [9] to [16]),
(iii) in those combinations in which the first primer is consisting of a partial sequence of SEQ ID NO: 3 and the second primer is consisting of a partial sequence of SEQ ID NO: 7 (combinations [17] to [24]), and
(iv) in those combinations in which the first primer is consisting of a partial sequence of SEQ ID NO: 4 and the second primer is consisting of a partial sequence of SEQ ID NO: 8 (combinations [25] to [28]),
   10³ copies/5 µL of the standard RNA were detected within 20 minutes for all combinations. Furthermore, fluorescence intensity ratios did not exceed 1.2 even after 30 minutes from the start of the reaction in a control test group (which were measured by adding RNA diluent to the reaction solution instead of standard RNA).

According to these results, it was indicated that by carrying out an RNA amplification reaction using:
(i) combinations in which the first primer is an oligonucleotide consisting of at least 15 contiguous bases in the base sequence listed as SEQ ID NO: 1 and the second primer is an oligonucleotide consisting of at least 15 contiguous bases in the base sequence listed as SEQ ID NO: 5,
(ii) the first primer is an oligonucleotide consisting of at least 15 contiguous bases in the base sequence listed as SEQ ID NO: 2 and the second primer is an oligonucleotide consisting of at least 15 contiguous bases in the base sequence listed as SEQ ID NO: 6,
(iii) combinations in which the first primer is an oligonucleotide consisting of at least 15 contiguous bases in the base sequence listed as SEQ ID NO: 3 and the second primer is an oligonucleotide consisting of at least 15 contiguous bases in the base sequence listed as SEQ ID NO: 7, or
(iv) combinations in which the first primer is an oligonucleotide consisting of at least 15 contiguous bases in the base sequence listed as SEQ ID NO: 4 and the second primer is an oligonucleotide consisting of at least 15 contiguous bases in the base sequence listed as SEQ ID NO: 8,
   CK19 RNA is detected more rapidly in comparison with detection of CK19 mRNA by the most commonly used RT-PCR method of the prior art (normally requiring 2 hours or more).

### Example 4 Measurement of CK19 RNA (Part 2)

Standard RNA having a lower concentration than that of Example 3 was measured using combinations of oligonucleotides of the present invention.

### (1) Experiment Method

Measurements were carried out using the same method as Example 3 with the exception of changing the combinations of oligonucleotides and RNA samples to that indicated below.

Combinations of oligonucleotides:
Combinations [5] to [8], [10], [12], [14], [16], [20] to [22], [24] and [27] to [30] shown in Table 1 were used.

RNA samples:
The standard RNA prepared in Example 1 was diluted with the RNA diluent used in part (1) of Example 3 to 50 copies/5 µL, 10² copies/5 µL and 10³ copies/5 µL.

### (2) Experiment Results

Designating the time of addition of enzyme as 0 minutes, samples were judged to be positive when the fluorescence intensity ratio of the reaction solution (fluorescence intensity value at a prescribed time ÷ background fluorescence intensity value) exceeded 1.2, and results showing the elapsed times at that time are shown in Table 3 as detection times.

**Table 3**

| Oligonucleotide combinations | Detection time (min) at each reference RNA concentration (copies/test) | | |
|---|---|---|---|
| | 50 copies | 10² copies | 10³ copies |
| [5] | 17.89 | 16.21 | 13.00 |
| [6] | 11.29 | 10.79 | 9.48 |
| [7] | 13.17 | 11.34 | 9.91 |
| [8] | 10.71 | 9.52 | 7.83 |
| [10] | 13.95 | 12.36 | 10.30 |
| [12] | 15.59 | 12.37 | 10.64 |
| [14] | 14.65 | 14.06 | 9.68 |
| [16] | 19.63 | 17.32 | 11.59 |
| [20] | 19.92 | 16.29 | 14.16 |
| [21] | 14.88 | 13.28 | 9.11 |
| [22] | 15.12 | 13.89 | 11.16 |
| [24] | 15.31 | 14.93 | 11.31 |
| [27] | 16.38 | 16.18 | 11.58 |
| [28] | 14.59 | 13.54 | 11.17 |
| [29] | 17.08 | 12.74 | 11.34 |
| [30] | 10.89 | 9.86 | 8.70 |

Standard RNA at a concentration of 50 copies/5 µL were detected within 20 minutes for all combinations of oligonucleotides examined in this experiment. On the basis of these results, CK19 mRNA was demonstrated to be able to be detected rapidly even at a low concentration of 50 copies/5 µL by amplifying CK19 mRNA using a combination of oligonucleotides of the present invention.

### Example 5 Evaluation of Specificity

A study was conducted of cross-reactivity with other cytokeratin RNA in a CK19 RNA amplification and detection system using combinations of oligonucleotides of the present invention.

### (1) Preparation of Cytokeratin 18 (CK18) RNA and Cytokeratin 20 (CK20) RNA

(1-1) Double-stranded DNA was cloned consisting of bases from positions 110 to 1434 (1325 bases) of a CK18 base sequence registered with GenBank (GenBank Accession No. NM_000224, 1485 bases), and bases from positions 40 to 1710 (1671 bases) of a CK20 base sequence registered with GenBank (GenBank Accession No. NM_019010, 1817 bases).
(1-2) In vitro transcription was carried out using the double-stranded DNA prepared in (1-1) as templates. Continuing, RNA was prepared after completely digesting the double-stranded DNA by treating with DNase I and purifying. The RNA was quantified by measuring absorbance at 260 nm.
(1-3) The standard RNA prepared in (1-2) were diluted using an RNA diluent (10 mM Tris-HCl buffer (pH 8.0), 0.1 mM EDTA, 0.5 U/µL ribonuclease inhibitor, 5.0 mM DTT) to 10¹⁰ copies/5 µL.

### (2) Experiment Method

Measurements were carried out using the same method as Example 3 with the exception of changing the combinations of oligonucleotides and RNA samples to that indicated below.

Combinations of oligonucleotides:
Combinations [8], [10], [21] and [28] shown in Table 1 were used.

Furthermore, combination [8] is equivalent to a combination in which the first primer is consisting of a partial sequence of SEQ ID NO: 1 and the second primer is consisting of a partial sequence of SEQ ID NO: 5,
combination [10] is equivalent to a combination in which the first primer is consisting of a partial sequence of SEQ ID NO: 2 and the second primer is consisting of a partial sequence of SEQ ID NO: 6,
combination [21] is equivalent to a combination in which the first primer is consisting of a partial sequence of SEQ ID NO: 3 and the second primer is consisting of a partial sequence of SEQ ID NO: 7, and
combination [28] is equivalent to a combination in which the first primer is consisting of a partial sequence of SEQ ID NO: 4 and the second primer is consisting of a partial sequence of SEQ ID NO: 8.

RNA samples:
The RNA sample used in Example 3 was used for CK19, and the samples prepared in (1) of the present example were used for CK18 and CK20.

### (3) Experiment Results

Designating the time of addition of enzyme as 0 minutes, samples were judged to be positive when the fluorescence intensity ratio of the reaction solution (fluorescence intensity value at a prescribed time ÷ background fluorescence intensity value) exceeded 1.2, and results showing the elapsed times at that time are shown in Table 4 as detection times. Furthermore, N.D. in Table 4 indicates samples for which the fluorescence intensity ratio at 30 minutes after enzyme addition was less than 1.2 (negative result).

**Table 4**

| Oligonucleotide combinations | Detection time (min) at each standard RNA concentration (copies/test) | | |
|---|---|---|---|
| | CK19 10³ copies | CK18 10¹⁰ copies | CK20 10¹⁰ copies |
| [8] | 8.67 | N.D. | N.D. |
| [10] | 10.14 | N.D. | N.D. |
| [21] | 9.86 | N.D. | N.D. |
| [28] | 11.34 | N.D. | N.D. |

Only CK19 RNA was detected for all of the combinations of oligonucleotides examined, while on the other hand, CK18 and CK20 were not detected despite using extremely large amounts of RNA samples of 10¹⁰ copies/5 µL. On the basis of these results, the CK19 RNA amplification and detection system using combinations of oligonucleotides of the present invention was demonstrated to specifically detect CK19 among other cytokeratins.

### Example 6 Measurement of CK19 RNA (Part 3)

The relationship between detection time and initial amount of standard RNA was confirmed by measuring various concentrations of standard RNA using combinations of oligonucleotides of the present invention.

### (1) Experiment Method

Measurements were carried out using the same method as Example 3 with the exception of changing the combinations of oligonucleotides and RNA samples to that indicated below.

Combinations of oligonucleotides:
Combinations [8], [10] and [30] of Table 1 were used.

RNA samples:
The standard RNA prepared in Example 1 was diluted using the RNA diluent used in part (1) of Example 3 to 10² copies/5 µL, 10³ copies/5 µL, 10⁴ copies/5 µL and 10⁵ copies/5 µL.

### (2) Experiment Results

Designating the time of addition of enzyme as 0 minutes, samples were judged to be positive when the fluorescence intensity ratio of the reaction solution (fluorescence intensity value at a prescribed time ÷ background fluorescence intensity value) exceeded 1.2, and results showing the elapsed times at that time are shown in Table 5 as detection times, while the results of plotting calibration curves based on the results of Table 5 are shown in FIG. 1. In addition, the fluorescence intensity ratios 20 minutes after the start of the reaction when the RNA sample measured at 10² copies/5 µL are also shown in Table 5.

**Table 5**

| Oligonucleotide combinations | Detection time (min) at each standard RNA concentration (copies/test) | | | | Fluorescence intensity ratio |
|---|---|---|---|---|---|
| | 10² copies | 10³ copies | 10⁴ copies | 10⁵ copies | |
| [8] | 9.07 | 8.35 | 7.54 | 6.89 | 1.62 |
| [10] | 11.30 | 10.08 | 9.15 | 8.02 | 2.40 |
| [30] | 10.41 | 9.14 | 8.23 | 7.33 | 1.81 |

All concentrations of the measured standard RNA were detected within 15 minutes after enzyme addition for all combinations of oligonucleotides examined in this experiment. In addition, when detection time was plotted on the vertical axis and the initial amount of standard RNA (represented as a log value of the number of copies) was plotted on the horizontal axis, detection times were dependent on the initial amount of standard RNA starting in a low copy region of 10² copies, and the calibration curves were able to be approximated to linear first order curves. Namely, it was indicated that by measuring CK19 mRNA according to the method of the present invention for an unknown sample and applying the resulting detection time to the calibration curves shown in FIG. 1, the amount of CK19 mRNA contained in the unknown sample can be estimated.

### Example 7 Measurement of CK19 RNA (Part 4)

CK19 mRNA present in a cultured cell extract derived from human pulmonary adenocarcinoma was quantified using combinations of oligonucleotides of the present invention.

### (1) Acquisition of Cultured Cells

Human pulmonary adenocarcinoma-derived cultured cells (PC-3) were acquired from the Health Science Research Resources Bank (HSRRB) (HSRRB No. JCRB0077).

### (2) Culturing of Cultured Cells

The PC-3 cells were cultured according to the media and culturing conditions described in the HSRRB web site (http://cellbank.nibio.go.jp/celldata/jcrb0077.htm).

### (3) Isolation of Lymphocytes

Blood (whole blood) was collected from healthy individuals and lymphocytes were isolated using Ficoll-Paque PLUS (GE Healthcare Life Sciences) in accordance with the manual provided.

### (4) Extraction of RNA

The number of cultured PC-3 cells was measured, prepared to cell concentrations of 5 cells, 50 cells, 5 × 10² cells and 5 × 10³ cells, and samples were prepared consisting of only the above cells or mixtures of the above cells with lymphocytes at 5 × 10⁶ cells followed by extracting RNA using the RNeasy Mini Kit (Qiagen) in accordance with the manual provided with the kit. Furthermore, the amount of the RNA extraction sample was 50 µL.

### (5) Measurement of CK19 RNA

Measurements were carried out using the same method as Example 3 with the exception of changing the combinations of oligonucleotides and RNA samples to that indicated below.

Combination of oligonucleotides:
Combination [10] of Table 1 was used.

RNA samples:
The RNA extraction sample described in (4) of the present example was diluted 10-fold using the RNA diluent described in (1) of Example 3 and 5 µL aliquots thereof was used.

### (6) Experiment Results

Designating the time of addition of enzyme as 0 minutes, samples were judged to be positive when the fluorescence intensity ratio of the reaction solution (fluorescence intensity value at a prescribed time ÷ background fluorescence intensity value) exceeded 1.2, and results showing the elapsed times at that time are shown in Table 6 (RNA extraction sample from PC-3 cells) and Table 7 (RNA extraction sample from mixture of PC-3 cells and lymphocytes) as detection times, while the results of calculating the amount of CK19 RNA contained in each RNA sample based on the detection times of Tables 6 and 7 and the calibration curve obtained in Example 6 (FIG. 1(b)) are shown in Table 8 (RNA extraction sample from PC-3 cells) and Table 9 (RNA extraction sample from mixture of PC-3 cells and lymphocytes).

**Table 6**

| Oligonucleotide combination | Detection time (min) of cultured cell samples (cells) | | | |
|---|---|---|---|---|
| | 5 cells | 50 cells | 5 × 10² cells | 5 × 10³ cells |
| [10] | 10.50 | 9.04 | 8.11 | 7.24 |

**Table 7**

| Oligonucleotide combination | Detection time (min) of cultured cell samples (cells/5 × 10⁶ lymphocytes) | | | |
|---|---|---|---|---|
| | 5 cells | 50 cells | 5 × 10² cells | 5 × 10³ cells |
| [10] | 12.59 | 9.40 | 8.27 | 7.28 |

**Table 8**

| Oligonucleotide combination | Amount of CK19 (copies) in cultured cell samples (cells) | | | |
|---|---|---|---|---|
| | 5 cells | 50 cells | 5 × 10² cells | 5 × 10³ cells |
| [10] | 500 | 11344 | 82850 | 532220 |

**Table 9**

| Oligonucleotide combination | Amount of CK19 (copies) in cultured cell samples (cells/5 × 10⁶ lymphocytes) | | | |
|---|---|---|---|---|
| | 5 cells | 50 cells | 5 × 10² cells | 5 × 10³ cells |
| [10] | 6 | 5254 | 58848 | 488597 |

The amounts of CK19 mRNA in each of the RNA samples determined from the calibration curve were nearly proportional to the number of PC-3 cultured cells used for both RNA extraction samples from cultured PC-3 cells and RNA extraction samples from mixtures of the cells with lymphocytes, thereby demonstrating that the CK19 RNA amplification and detection method using oligonucleotides of the present invention is concentration-dependent.

### Example 8 Base Sequence Analysis of CK19 RNA

### Amplification Product

The base sequences were analyzed for the double-stranded DNA contained in the samples obtained following the nucleic acid amplification reaction in Example 7. As a result of analyzing the base sequences, corresponding base sequences yielded by CK19 mRNA amplification were confirmed for all samples by using each of the combinations of oligonucleotides.

In other words, this indicates that CK19 RNA is amplified and detected in the present invention, and not other non-specific RNA. Although contaminating RNA other than CK19 RNA was included in the RNA extract used in Example 7, the measurement method of the present invention only amplified and detected CK19 mRNA, and can be said to be extremely specific. In addition, since extracts were able to be measured dependent on the number of cultured cells used and from extremely small amounts of cultured cells (5 cells) even in the presence of large amounts of contaminants (RNA other than CK19 mRNA), the method can be said to have extremely high sensitivity as well as superior accuracy.

On the basis of the above, the method of measuring CK19 mRNA of the present invention enables CK19 mRNA contained in an unknown sample to be measured specifically, rapidly and with high sensitivity, and was also indicated to enable quantification by using a calibration curve.

### INDUSTRIAL APPLICABILITY

According to the present invention, cytokeratin 19 (CK19) RNA can be amplified specifically, rapidly and with high sensitivity in a single step under comparatively low and constant temperature conditions (40 to 60°C, and preferably 43°C), or amplification products thereof can be detected directly.

## Claims

1. A method of detecting cytokeratin 19 (CK19) mRNA in a sample employing an RNA amplification process, said method consisting of the following steps :
(1) synthesizing a cDNA with an enzyme having RNA-dependent DNA polymerase activity, by using CK19 mRNA as a template, and a primer consisting of the oligonucleotide listed as SEQ ID NO: 33, thereby producing a double-stranded RNA-DNA;
(2) decomposing the RNA of said double-stranded RNA-DNA obtained in (1) above by use of an enzyme having ribonuclease H (RNase H) activity, thereby forming a single-stranded DNA complementary to said specific base sequence;
(3) forming double-stranded DNA with an enzyme having DNA-dependent DNA polymerase activity, using a primer consisting of the oligonucleotide listed as SEQ ID NO: 23 and that includes an RNA polymerase promoter sequence at the 5'-end thereof, and said single-stranded DNA obtained in (2) as a template;
(4) forming an RNA transcription product derived from said specific base sequence with an enzyme having RNA polymerase activity, using said double-stranded DNA having said promoter sequence obtained in (3) above as template;
(5) allowing said RNA transcription product derived from said specific base sequence obtained in (4) to serve as a template for the synthesis of a new double-stranded DNA by the first and second primers, and allowing said reactions (1) to (4) to proceed to cause an RNA amplification in a chain reaction to obtain an amplified RNA transcription product derived from said specific base sequence and,
(6) detecting said amplified RNA transcription product by detecting a change in fluorescent properties in the presence of a fluorescent dye-labeled oligonucleotide probe designed to change its fluorescent properties when it forms a complementary double-strand with the target RNA.

2. The detection method according to claim 1, **characterized in that** the fluorescent dye-labeled oligonucleotide probe is an intercalating fluorescent dye-labeled oligonucleotide probe in which an intercalating fluorescent dye is bound through a linker.

3. The detection method according to claim 2, **characterized in that** the intercalating fluorescent dye-labeled oligonucleotide probe contains an oligonucleotide consisting of the base sequence listed as SEQ ID NO: 43.

4. The method of detecting CK19 mRNA according to any of claims 1 to 3, **characterized in that** prior to the synthesizing step described in (1), a step is carried out for cleaving the RNA at the 5'-end site of the specific base sequence by use of a specific base sequence in CK19 mRNA as a template, and using :
(i) a cleaving oligonucleotide having a region that overlaps with the 5'-end site of a region homologous to the first primer in the specific base sequence, and a sequence complementary to an adjacent region on the 5' side from the site, said cleaving oligonucleotide consisting of the base sequence listed as SEQ ID NO: 13, and
(ii) an enzyme having ribonuclease H (RNase H) activity.

## Patentansprüche

1. Verfahren zum Nachweisen von Cytokeratin 19 (CK19)-mRNA in einer Probe unter Einsatz eines RNA-Amplifikationsprozesses, wobei das Verfahren aus den folgenden Schritten besteht:
(1) Synthetisieren einer cDNA mit einem Enzym mit RNAabhängiger DNA-Polymeraseaktivität durch Verwenden von CK19-mRNA als Matrize und eines Primers, der aus dem Oligonucleotid besteht, das als SEQ ID NO: 33 verzeichnet ist, um dadurch eine doppelsträngige RNA-DAN zu erzeugen;
(2) Abbauen der RNA der zuvor in (1) erhaltenen doppelsträngigen RNA-DNA durch Verwendung eines Enzyms mit Ribonuclease H (RNase H)-Aktivität, um dadurch eine einzelsträngige DNA zu bilden, die komplementär zur spezifischen Basensequenz ist;
(3) Bilden einer doppelsträngigen DNA mit einem Enzym mit DNA-abhängiger DNA-Polymeraseaktivität unter Verwendung eines Primers, der aus dem Oligonucleotid besteht, das als SEQ ID NO: 23 verzeichnet ist und das eine RNA-Polymerase-Promotorsequenz am 5'-Ende davon umfasst, und der in (2) erhaltenen einzelsträngigen DNA als Matrize;
(4) Bilden eines RNA-Transkriptionsprodukts, das von der spezifischen Basensequenz abgeleitet ist, mit einem Enzym mit RNA-Polymeraseaktivität unter Verwendung der zuvor in (3) erhaltenen doppelsträngigen DNA mit der Promotorsequenz als Matrize;
(5) Zulassen, dass das in (4) erhaltene RNA-Transkriptionsprodukt, das von der spezifischen Basensequenz abgeleitet ist, als Matrize für die Synthese einer neuen doppelsträngigen DNA durch die ersten und zweiten Primer dient, und Zulassen, dass die Reaktionen (1) bis (4) fortschreiten, um eine RNA-Amplifikation in einer Kettenreaktion zu bewirken, um ein amplifiziertes RNA-Transkriptionsprodukt zu erhalten, das von der spezifischen Basensequenz abgeleitet ist, und
(6) Nachweisen des amplifizierten RNA-Transkriptionsprodukts durch Nachweisen einer Änderung von Fluoreszenzeigenschaften in Gegenwart einer mit fluoreszierendem Farbstoff markierten Oligonucleotid-Sonde, die so ausgelegt ist, dass sie ihre Fluoreszenzeigenschaften ändert, wenn sie einen komplementären Strang mit der Ziel-RNA bildet.

2. Nachweisverfahren nach Anspruch 1, **dadurch gekennzeichnet, dass** die mit fluoreszierendem Farbstoff markierte Oligonucleotid-Sonde eine mit interkalierendem fluoreszierendem Farbstoff markierte Oligonucleotid-Sonde ist, in welcher ein interkalierender fluoreszierender Farbstoff durch einen Linker gebunden wird.

3. Nachweisverfahren nach Anspruch 2, **dadurch gekennzeichnet, dass** die mit interkalierendem fluoreszierendem Farbstoff markierte Oligonucleotid-Sonde ein Oligonucleotid enthält, das aus der Basensequenz besteht, die als SEQ ID NO: 43 verzeichnet ist.

4. Verfahren zum Nachweisen von CK19-mRNA nach einem der Ansprüche 1 bis 3, **dadurch gekennzeichnet, dass** vor dem in (1) beschriebenen Syntheseschritt ein Schritt zum Spalten der RNA an der 5'-Endstelle der spezifischen Basensequenz durch Verwendung einer spezifischen Basensequenz in CK19-mRNA als Matrize und unter Verwendung
(i) eines spaltenden Oligonucleotids mit einer Region, die sich mit der 5'-Endstelle einer Region überlappt, die homolog zum ersten Primer in der spezifischen Basensequenz ist, und einer Sequenz, die komplementär zu einer benachbarten Region an der 5'-Seite von der Stelle ist, wobei das spaltende Oligonucleotid aus der Basensequenz besteht, die als SEQ ID NO: 13 verzeichnet ist; und
(ii) eines Enzyms mit Ribonuclease H (RNase H)-Aktivität ausgeführt wird.

## Revendications

1. Procédé de détection d'ARNm de cytokératine 19 (CK19) dans un échantillon en utilisant un procédé d'amplification d'ARN, ledit procédé consiste en les étapes suivantes:
(1) synthétiser un ADNc avec une enzyme ayant une activité ADN polymérase ARN-dépendante, en utilisant l'ARNm de CK19 en tant que matrice, et une amorce constituée de l'oligonucléotide énuméré comme étant la séquence SEQ ID NO : 33, produisant ainsi un ARN-ADN double brin ;
(2) à décomposer l'ARN dudit ARN-ADN double-brin obtenu dans (1) ci-dessus par l'utilisation d'une enzyme ayant une activité ribonucléase H (RNase H), formant ainsi un ADN simple brin complémentaire de ladite séquence de bases spécifique ;
(3) à former l'ADN double brin
avec une enzyme ayant une activité ADN polymérase ADN-dépendante, en utilisant une amorce constituée de l'oligonucléotide énuméré comme étant la séquence SEQ ID NO : 23
et qui comporte une séquence promotrice d'ARN polymérase au niveau de son extrémité 5', et ledit ADN simple brin obtenu dans (2) en tant que matrice ;
(4) à former un produit de transcription d'ARN dérivé de ladite séquence de bases spécifique avec une enzyme ayant une activité ARN polymérase, en utilisant ledit ADN double brin ayant ladite séquence promotrice obtenue dans (3) ci-dessus en tant que matrice ;
(5) à permettre audit produit de transcription d'ARN dérivé de ladite séquence de bases spécifique obtenue dans (4) de servir de matrice pour la synthèse d'un nouvel ADN double brin par les première et deuxième amorces, et à permettre auxdites réactions (1) à (4) de procéder afin de provoquer une amplification d'ARN dans une réaction en chaîne pour obtenir un produit de transcription d'ARN amplifié dérivé de ladite séquence de bases spécifique et,
(6) à détecter ledit produit de transcription d'ARN amplifié en détectant une modification des propriétés fluorescentes en présence d'une sonde oligonucléotidique marquée avec un colorant fluorescent conçue pour modifier ses propriétés fluorescentes lorsqu'elle forme un double-brin complémentaire avec l'ARN cible.

2. Procédé de détection selon la revendication 1, **caractérisé en ce que** la sonde oligonucléotidique marquée avec un colorant fluorescent est une sonde oligonucléotidique marquée avec un colorant fluorescent intercalant où un colorant fluorescent intercalant est lié par l'intermédiaire d'un lieur.

3. Procédé de détection selon la revendication 2, **caractérisé en ce que** la sonde oligonucléotidique marquée avec un colorant fluorescent intercalant contient un oligonucléotide constitué de la séquence de bases énumérée comme étant la séquence SEQ ID NO : 43.

4. Procédé de détection d'ARNm de CK19 selon l'une des revendications 1 à 3, **caractérisé en ce qu'**avant l'étape de synthèse décrite dans (1), une étape est réalisée pour cliver l'ARN au niveau du site d'extrémité 5' de la séquence de bases spécifique par utilisation d'une séquence de bases spécifique dans ARNm de CK19 en tant que matrice, et en utilisant :
(i) un oligonucléotide de clivage ayant une région qui chevauche le site d'extrémité 5' d'une région homologue à la première amorce dans la séquence de bases spécifique, et une séquence complémentaire à une région adjacente sur le côté 5' du site, ledit oligonucléotide de clivage constitué de la séquence de bases énumérée comme étant la séquence SEQ ID NO _{:} 13, et
(ii) une enzyme ayant une activité ribonucléase H (RNase H).
